# EUROPEAN PATENT APPLICATION

(11) **EP 4 516 214 A1**
(43) Date of publication of application: **05.03.2025**
(21) Application number: 23195015.5
(22) Date of filing: 04.09.2023
(51) Int. Cl.: A61B 5/0215, A61B 5/07, G16H 40/67, G16H 50/20, A61B 5/01, A61B 5/11

(54) **IMPLANTABLE PRESSURE SENSOR FOR CONTINUOUS MONITORING OF THE SYSTEMIC ARTERIAL BLOOD PRESSURE**

(71) Applicant: BIOTRONIK SE & Co. KG, 12359 Berlin (DE)
(72) Inventor: Arndt, Andreas, 12555 Berlin (DE); Wegerich, Franziska, 12055 Berlin (DE); Skerl, Olaf, 18209 Bad Doberan (DE); Moß, Christian, 14612 Falkensee (DE)
(74) Representative: Biotronik Corporate Services SE

(57) **Abstract**

The present disclosure relates to methods and systems for continuously monitoring an arterial blood pressure of a patient as well as methods and systems for assisting patients having hypertension. A method for continuously monitoring an arterial blood pressure of a patient may comprise: Determining (120) arterial blood pressure values of the patient by an implantable pressure sensor (210) for a plurality of times per day; forwarding (140) the arterial blood pressure values to an external device (220); accounting for changes of the barometric pressure by the external device and indicating (160), by the external device, blood pressure values (264) at least in part based on the arterial blood pressure values.

## Description

The present invention generally relates to an implantable pressure sensor for continuous monitoring of systemic arterial blood pressure and a system comprising such a sensor. Moreover, it relates to corresponding monitoring as well as systems. Further, the present invention relates to corresponding systems and methods for assisting patients with hypertension.

There are various diseases associated with irregular blood pressure values. Hypertension, for example, is a particular risk factor for potentially lethal secondary diseases such as arteriosclerosis, cardiovascular accidents, heart failure, valvular heart disease, vascular dementia, etc. These diseases increase mortality, reduce the quality of life, and create significant costs for treating or generally coping with these diseases. Treating hypertension may significantly reduce the risk of such secondary diseases.

The treatment of hypertension usually involves medication and is rather sophisticated. Typically, a varying titration of the medication (as for example required for diuretics to treat heart failure) may not be required frequently. But adequately determining the right dose and, in particular, regular intake of the medication is paramount. However, there is frequently only limited blood pressure data to find the right dose. Another issue is patient compliance. Hypertension patients typically feel well (despite their condition) such it is difficult to ensure patient compliance in taking the medication and/or acquiring blood pressure values.

When measuring blood pressure data by external devices to find the right dose, typically sufficient accuracy is not provided. For example, devices based on arterial pressure measurement by photoplethysmography rely on pulse train transit times or morphology evaluation that are based on an assumed constant impedance of the vascular system. However, this impedance in fact varies over time, such that these devices cannot accurately measure arterial blood pressure on a long-term basis. Apart from that, they are unable to detect systolic overshoots of the aortic pressure caused by reflections. Other external devices that, for example, determine blood pressure at the wrist of a patient are particularly imprecise during atrial fibrillation which occurs frequently in hypertension patients. Moreover, if the patient does not accurately position the wrist at the height of the heart, such measurements are inherently imprecise, such that accurate measurements are only obtained in case of strict patient compliance.

There are also implantable blood pressure sensors that may determine left or right atrial pressure or related pressure values such as pulmonary artery pressure. However, these pressure values are of limited use when it comes to hypertension which is best assessed based on systemic arterial blood pressure. Moreover, known devices anyway are only able to determine pressure values on an infrequent basis up to once a day, which does not allow a tight monitoring of blood pressure. Another issue with known implantable sensors is that they typically rely on external equipment. For example, they may only provide passive communication, which would require the patient to carry dedicated external equipment at all times in order to access measured blood pressure values.

There is therefore a need to improve the monitoring and treatment of patients (at risk of) suffering from hypertension.

This need is at least partly met by the aspects as described herein.

According to a first aspect, a method for continuously monitoring an arterial blood pressure of a patient is provided. The method may comprise determining arterial blood pressure values of the patient by an implantable pressure sensor for a plurality of times per day. It may further comprise forwarding the arterial blood pressure values to an external device and indicating, by the external device, blood pressure values at least in part based on the arterial blood pressure values.

For example, the arterial blood pressure values may be determined on a predetermined basis at least three times per day, at least six times per day or at least 12 times per day or every hour or even more frequently, such as two or four times per hour (the listed frequencies may pertain to at least one day or also to each day).

Hence, it is ensured that the arterial blood pressure can be accurately monitored in a long-term stable and a closely meshed manner, e.g. by an intravascular sensor that can be implanted in a minimally invasive manner and that measures the systemic pressure in-situ, taking into account the potential strong variations encountered in the course of time. This may particularly be beneficial for hypertension patients. Not only may a medication dose most suitable for a specific hypertension patient be accurately determined based on the indicated blood pressure values. Also, it may be possible to verify patient compliance in taking the medication, e.g. by verifying whether the monitored pressure values are as expected. Hence, the medication for hypertension patients may be dosed more accurately right from the outset. Further, patient compliance may be ensured during ongoing medication, and countermeasures may be taken in case of non-compliant patients before they experience serious symptoms due to their non-compliance.

The implantable sensor may particularly be configured to determine the arterial blood pressure (at least once per day) during the night-time and (at least once per day) during the daytime, and/or with high patient activity and with low patient activity. This may be achieved by configuring the implantable sensor to measure at predetermined times, such that suitable data is automatically obtained, without having to rely on patient compliance. For example, the implantable sensor may comprise a configurable timer, wherein the timer may trigger measurements at the configured times, or in the configured intervals, for example.

For example, the systemic arterial pressure may be determined by an implantable sensor implanted in an aorta of a patient, such as an aorta descendens or an aorta abdominalis. The implantable sensor may be an active sensor programmed to automatically determine the arterial blood pressure values a plurality of times per day, e.g. based on a predetermined timing, such as every 12, 8, 4, 2 or 1 hours, or even more frequently such as 2 or 4 times per hour or even every minute. This may ensure that pressure values are determined during different patient states (e.g. whether or not the patient is exercising or sleeping), such that blood pressure values are obtained for various patient states (e.g. at varying activity states) that may all be taken into account for counteracting hypertension in a tailored manner.

The indicated blood pressure values may, for example, correspond to the received arterial blood pressure values. But the external device may also perform algorithmic processing on the received arterial blood pressure values such that correspondingly processed blood pressure values may be indicated. For example, such processing may account for variations in barometric pressure values. Also, the values may be processed for improved visualization (e.g. to indicate a trend of values over time, including interpolated values, etc.).

It is noted that an implantable sensor may relate to a sensor that is suitable for being implanted into a patient, but not yet implanted, but it may also relate to a sensor that has already been implanted.

The external device may relate to a patient device that is in wireless communication with the implantable sensor. For example, it may receive the arterial blood pressure values via Bluetooth (e.g. Bluetooth Low Energy), WLAN, Medical Implant Communication Service (MICS), etc. (e.g. directly from the implantable sensor or via an implantable or an external relay device).

The external device may preferably be a smartphone, tablet, or similar device. It may also be a dedicated device specifically dedicated to communicating with the implantable sensor. In either case, the external device may comprise a corresponding application or program that implements the method steps as outlined herein. Such an external device may also be a separate aspect of the present invention independently from the aspects described herein with reference to other devices.

The method may further comprise adapting the arterial blood pressure values at least in part based on barometric pressure values. For example, barometric pressure changes may be taken into account to correct the absolute arterial blood pressure values received from the implantable sensors such that variations solely due to barometric pressure changes may be removed. Hence, the relative blood pressure is obtained.

The barometric pressure values may be determined by the external device. For example, the external device (e.g. a smartphone) may comprise a barometric pressure sensor for acquiring the barometric pressure values. Hence, without needing further equipment or communications, the external device may directly adapt the received arterial blood pressure values based on corresponding barometric pressure values (e.g. relating to the same or similar times). To ensure that barometric pressure values are available for times in the past (the arterial blood pressure values may be received with a delay at the external device, for example), the external device may be configured to continuously monitor the barometric pressure (e.g. based on a similar predetermined timing that is used by the implantable pressure sensor) and associate the respective values with corresponding time stamps.

The method may further comprise the step of determining the proximity between the patient and the external device at the time of the determination of the barometric pressure value by the external device. The accuracy of the determination of the relative blood pressure (absolute arterial blood pressure corrected by the barometric pressure) depends on the deviation between the measured barometric pressure and the barometric pressure experienced by the patient. Ideally the patient experiences the same barometric pressure as the device (e.g. the external device) determining the barometric pressure. One way to ensure this is to determine whether the external device is in the proximity of the patient at the time of the determination of the barometric pressure.

The step of determining the proximity between the patient and the external device at the time of the determination of the barometric pressure may comprise at least one of the following:
- comparing the motion profiles of the implant and the external device
- Signal exchange between implant and external device prior, during or after the determination of the barometric pressure

The implant and the external device may comprise an accelerometer. The data derived from the accelerometer may be stored at the implant and at the external device. The data of the accelerometers may be used to create a motion profile for the implant (and therefor the patient) and for the external device. The motion profile of the external device may be compared to the motion profile of the implant. If the two motion profiles match or if the two motion profiles are similar, it could be assumed that the external device was with the patient and experienced therefor the same barometric pressure as the patient.

The implant and the external device may exchange a signal in connection with the determination of the pressure (prior/during/after the determination of the arterial blood pressure/barometric pressure). The signal may be just a short ping ("are you there?") from the external device to the implant or vice versa and a corresponding response signal. Such a short ping signal would be energy efficient and no burden for the longevity of the implant battery. If the external device is in range for a communication with the implant, it could be assumed that the external device was close enough to the patient to experience the same barometric pressure.

Both above disclosed steps for determining the proximity between the patient and the external device could be used separately or in combination.

The method may further comprise the step of determining patient temperature values and/or patient's physical activity and posture values by measuring the acceleration in the implantable pressure sensor for a plurality of times per day. Moreover, it may further comprise forwarding the determined patient temperature values and/or patient acceleration values to the external device. For example, the implantable pressure sensor may comprise a temperature sensor and/or an accelerometer. Each may for example be configured to determine patient temperate (T) and/or acceleration (a) values synchronously with the determination of the arterial blood pressure values (p), e.g. based on the same predetermined timing. The corresponding data may be stored in the implant and forwarded to the external device as soon as a communication is established in the form of datasets with a time stamp (t), e.g. in the form of (p, T, a, t). The external device may be configured to indicate the temperature and/or acceleration values as well (e.g. as received from the sensor and/or algorithmically processed, e.g. similarly as outlined herein with respect to the pressure values), and these may then additionally be taken into account for handling hypertension patients. For example, the blood pressure values under physical stress (e.g. if the patient is exercising as indicated by high acceleration values) may specifically be taken into account, e.g., for determining an initial dose of a medication or for determining a deviation of the patient from an optimal state (e.g. due to non-compliance in taking the medication), etc.

The method may comprise the step of determining arterial blood pressure values at different activity levels of the patient. For example, this may be ensured by frequently determining the blood pressure in the course of the day as outlined herein (instead of measuring only once per day, e.g. at a predetermined night-time, or even less frequently) and, possibly, corresponding patient acceleration and/or patient temperature values. For example, different activity levels may be indicated by different acceleration and/or temperature values.

The method may further comprise forwarding, by the external device, pressure data based on the arterial blood pressure values to a remote system. For example, the external device may forward the received arterial pressure values. Also, it is possible that it is configured to forward the indicated pressure values that have been algorithmically processed as outlined herein. Optionally, the pressure data may be forwarded together with corresponding acceleration and/or temperature data of the patient. Hence, the remote system may be enabled to provide this data to further parties, e.g. medical staff, relatives, etc., that may access the data from the remote system, e.g. via a web interface or a corresponding application and/or program. Also, the data may be monitored by the remote system and, e.g. in case of irregular data, e.g. a strongly increasing blood pressure, medical staff and/or relatives may be alerted.

The method may, however, also comprise the step of receiving feedback at the external device from the remote system based on the pressure data. The feedback may optionally also be based on the optionally forwarded acceleration and/or temperature data of the patient. For example, the data may be analysed by the remote system and in case of need, feedback (such as an alarm or a recommended action) may be provided to the external device. Such feedback may however also be provided to other parties, e.g. medical staff and/or relatives.

The method may further comprise the step of indicating an alarm and/or a recommended action at least in part based on the arterial pressure values. For example, the alarm and/or recommended action may be determined by the external device based on processing the pressure data and then indicated by the external device. For example, a recommended action may be to carefully follow the prescribed medication in case the data indicates a slight pressure increase due to suspected omission of a medication dose. The patient may then follow this action and/or a physician may instruct the patient to follow this action.

The alarm and/or recommended action may further be based on the patient temperature values and/or patient acceleration values. For example, the external device itself may at least in part process the available temperature and/or acceleration data (possibly in addition to the pressure data) to derive a suitable recommended action and/or alarm.

The alarm and/or recommended action may further be based on the feedback received from the remote system. For example, the pressure data, and optionally the temperature and/or acceleration data (if forwarded to the remote system by the external device), may be analysed by the remote system and then a corresponding feedback, e.g. to indicate a certain alarm and/or recommended action, may be determined based thereon. For example, the external device may then indicate that alarm and/or recommended action.

The method may further comprise a step of comparing the determined arterial blood pressure values and/or barometric pressure values with a trend of previously and subsequently determined arterial blood pressure values and/or barometric pressure values. Such a step would increase the accuracy of the determined data. Each determined arterial blood pressure value, barometric pressure value and/or relative blood pressure value (based on the arterial pressure value corrected by the barometric pressure value) may be compared with the last determined pressure values and the subsequently determined pressure values, in particular with the value measured immediately before and after, in particular with the last 3 values measured immediately before and after, in particular with the last 5 values measured immediately before and after. If the determined value deviates by a certain threshold from the value measured immediately before and after and/or by a certain threshold from the trend of the values measured immediately before and after, such a determined pressure value will be ruled out as error and not used for the determination of the trend as well. The values acquired before (k-1, k-2, k-3,...) and after (k+1, k+2, k+3,...) the value at time instant k may also be used as input to a median filter of suitable filter length (typically 3 to 15) to cancel out outliers. A median filter sorts the set of acquired pressure values in ascending order and picks the middle value of the sorted set to be used as the determined pressure value at time instant k.

A second aspect relates to a system for continuously monitoring an arterial blood pressure of a patient. The system may comprise an implantable pressure sensor for determining arterial blood pressure values of the patient a plurality of times per day. It may further comprise an external patient device for receiving the arterial blood pressure values from the implantable pressure sensor. The external patient device may be configured to indicate blood pressure values at least in part based on the arterial blood pressure values. It may further comprise a remote system where the blood pressure values at least in part based on the arterial blood pressure values are displayed.

A third aspect relates to a system for assisting a patient having hypertension, e.g. for treating the hypertension of the patient. The system may comprise an implantable pressure sensor for determining arterial blood pressure values of the patient a plurality of times per day. It may further comprise an external patient device for receiving the arterial blood pressure values from the implantable pressure sensor. The external patient device may be configured to indicate an alarm and/or a recommended action to mitigate the hypertension at least in part based on the arterial blood pressure values.

This may allow a particularly effective counteracting of hypertension, since suitable (e.g. frequent and reliable) pressure measurements may be taken into account for determining whether an alarm is needed (e.g. in case of a sharp increase of pressure) or a certain action has to be recommended to the patient (e.g. a reminder to the patient to take the medication in case of a small deviation, e.g. that increases over time, of determined blood pressure from the target value).

The implantable pressure sensor may comprise an accelerometer and/or a temperature sensor. For example, based on accelerometer data, body position and activity of the patient may be determined (e.g. by the external device and/or a remote system).

The implantable sensor may be an active sensor. The implantable sensor may comprise a battery. The battery may be (wirelessly) rechargeable. The battery may provide sufficient power to implement the measurements as described herein.

A fourth aspect relates to a method for assisting a patient having hypertension, e.g. for treating the hypertension of the patient. The method may comprise determining arterial blood pressure values of the patient by an implantable pressure sensor a plurality of times per day. It may further comprise forwarding the arterial blood pressure values to an external device and/or a remote system. It may still further comprise indicating, by the external device and/or the remote system, an alarm and/or a recommended action to mitigate the hypertension at least in part based on the arterial blood pressure values.

Since the method does not itself affect the patient, it may also be referred to as a method for assisting a patient having hypertension without necessarily involving a treatment.

The method may further comprise forwarding pressure data at least in part based on the arterial blood pressure values (and optionally based on the temperature and/or acceleration values) to a remote system. The method may further comprise receiving feedback from the remote system, at least in part based on the forwarded pressure data (and optionally based on the temperature and/or acceleration values). The alarm and/or recommended action may at least in part be based on the feedback.

The method may further comprise determining the arterial blood pressure values at different activity levels of the patient.

Specifically, the method according to the fourth aspect may also comprise adapting the absolute arterial blood pressure values at least in part based on barometric pressure values (P), wherein the barometric pressure values may be determined by the external device.

It may further comprise determining patient temperature values and/or patient's activity and/or posture by measuring acceleration values by the implantable pressure sensor for a plurality of times per day. The determined patient temperature values and/or patient acceleration values may be forwarded to the external device.

It may comprise determining arterial blood pressure values at different activity levels of the patient.

The alarm and/or recommended action may additionally be based on the patient temperature values and/or patient acceleration values.

It is noted that the method steps of the first and fourth aspects may also be combined. All aspects described with respect to the method for continuously monitoring may also be applied to the method for assisting patients having hypertension, and vice versa. Also, a system may comprise aspects of the second and third aspects as described herein. For example, a single external device in communication with a single implantable blood pressure sensor may indicate an alarm and/or a recommended action to mitigate a hypertension at least in part based on the received arterial blood pressure values as well as it may indicate blood pressure values at least in part based on the arterial blood pressure values. All aspects described with reference to a system for continuously monitoring may also be applied to a system for assisting patients having hypertension, and vice versa.

Also, the aspects described herein with reference to method steps may be implemented as functions of the respective systems, even if this may, for brevity, not expressly be mentioned for each and every aspect. Similarly, all aspects described with reference to systems may equally be implemented as corresponding method steps.

Notably, the systems described herein may optionally include the remote system as described herein.

Aspects of the present disclosure will be described in more detail in the following by reference to the accompanying Figures.
- Fig. 1: shows an exemplary embodiment of a method for continuously monitoring an arterial blood pressure of a patient.
- Fig. 2: shows an exemplary embodiment of a system for continuously monitoring an arterial blood pressure of a patient and/or for assisting patients having hypertension.
- Fig. 3: shows an exemplary embodiment of a method for assisting patients having hypertension.

In the following, exemplary embodiments will be described in more detail, with reference to systems and methods. While specific feature combinations are described in the following with respect to exemplary embodiments, it is to be understood that the disclosure is not limited to such embodiments.

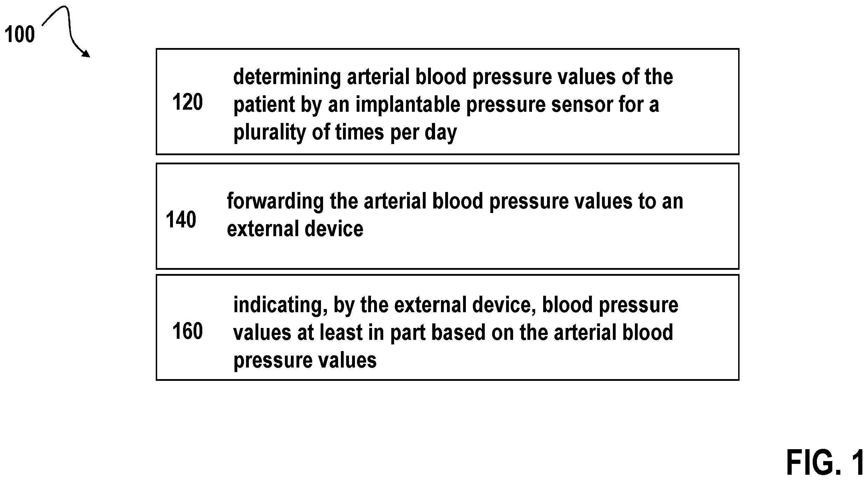

Figure 1 shows an exemplary method 100 for continuously monitoring an arterial blood pressure of a patient. In step 120, arterial blood pressure values (and optionally temperature and/or acceleration values) of the patient may be determined by an implantable pressure sensor for a plurality of times per day. In step 140, the arterial blood pressure values (and optionally the temperature and/or acceleration values) may be forwarded to an external device. In step 160, blood pressure values that are based at least in part on the arterial blood pressure values (and optionally temperature, acceleration, and/or body position, and/or activity data based on the temperature and/or accelerometer values) may be indicated by the external device. It is noted that the method needs not necessarily follow the sequence of steps 120, 140, 160 in a sequential manner. Instead, a first pressure value may be determined, forwarded, and indicated, for example, and the one or more further pressure values determined at a later time may be forwarded separately. Subsequently, the indication by the external device may be updated, based on the one or more further pressure values. This may enable to monitor the arterial blood pressure continuously, e.g. based on regular updates throughout the day. In some examples, the data acquired by the implantable sensor may be transmitted as soon as a connection to the external device is available (e.g. at night time), wherein, if the connection is not available, the data may be buffered by the sensor and then transmitted as soon as the connection becomes available again.

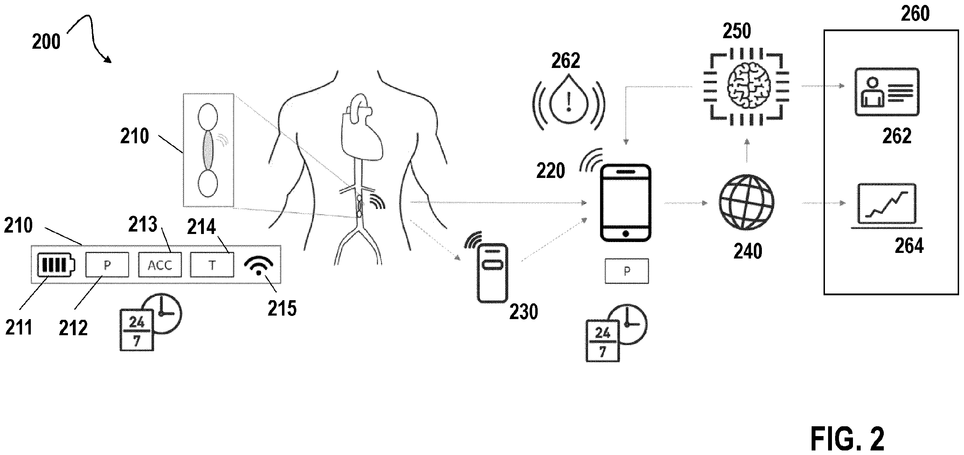

Figure 2 shows an exemplary system 200 for continuously monitoring an arterial blood pressure of a patient and/or for assisting patients having hypertension, e.g. for treating the hypertension of the patient.

System 200 comprises an implantable pressure sensor 210. It may be adapted to be implanted into a systemic arterial vascular system, e.g. an aorta abdominalis, of a patient, e.g. having hypertension. Implantable pressure sensor 210 may be configured to be implanted in a minimally invasive manner. Implantable pressure sensor 210 may be implanted using a delivery catheter. It may specifically be implanted without fluoroscopy. The exact implant position may be determined by an insertion depth of the delivery catheter. For verifying a suitable diameter of the vessel at the implantation site, it may be controlled via optical coherence tomography measurements that may be done by corresponding means integrated into the delivery catheter. Alternatively, a wedge-balloon at a tip of the delivery catheter may be used. The balloon may not (yet) be completely filled when introducing the sensor beyond the desired target position within the respective vessel. Prior to withdrawing the catheter (towards the desired target position), the balloon may be filled completely. It may then be withdrawn until it completely blocks the vessel, at which point the target diameter (and thus the desired target position) is reached.

Implantable pressure sensor 210 may comprise a pressure transducing unit 212 which allows determining systemic arterial pressure. Moreover, pressure sensor 210 may comprise an accelerometer 213 and/or a temperature sensor 214. It may further comprise a battery 211 to provide energy to implantable pressure sensor 210 to enable automatic or autarkic measurements. Additionally, it may comprise a transmitter 215. Transmitter 215 may optionally be implemented as a transceiver. Transmitter 215 may be configured to operate based on Bluetooth (e.g. Bluetooth Low Energy), WLAN, MICS, or any other type of intrabody communication.

Implantable pressure sensor 210 may be an active sensor (also in terms of sensing acceleration and/or temperature), and it may be configured to operate at any time, e.g. according to a predetermined schedule, e.g. in predetermined steps of time, e.g. every hour, every four hours, etc. Implantable pressure sensor 210 may comprise a processing unit (not shown in Fig. 2), such as a processor, microcontroller, ASIC, etc. It may be programmed to trigger pressure (and optionally acceleration and/or temperature) measurements at predetermined times, for example, such that, in principle, measurements may be provided at any time of the day or week (24/7). Implantable pressure sensor 210 may be programmed accordingly at manufacture, for example. Additionally or alternatively, it may be programmed by commands received via transceiver 215. Transmitter 215 may be configured to forward sensor values (e.g. arterial pressure values, patient temperature values and/or patient acceleration values) to an external device 220. Hence, pressure sensor 210 may allow not only determining the arterial pressure but also allow inferring the body position and/or physical activity of the patient (based on the accelerometer and/or temperature values).

In the example of Fig. 2, external device 220 is a patient device, in particular a handheld patient device, such as a smartphone or a tablet. External device 220 and transmitter 215 may be in direct communication. Additionally or alternatively, they may be in communication via a relay device 230. This may for example be helpful to facilitate transmitter 215 operating with MICS which may typically not be supported by smartphones or tablets, such that an external MICS device may be provided as relay device 230. Relay device 230 may then communicate with external device 220 via Bluetooth (e.g. Bluetooth Low Energy), for example.

External device 220 may comprise an ambient (or barometric) pressure sensor. It may be configured to acquire ambient pressure data P. It may be programmed to trigger pressure measurements at predetermined times, for example, such that, in principle, measurements may be provided at any time of the day or week (24/7). For example, it may trigger the pressure measurement with a timing adapted to that used by pressure sensor 210 for determining arterial pressure values.

External device 220 may be configured to adapt the received absolute arterial pressure values based on corresponding barometric pressure values. Moreover, the pressure data may be adapted for display as outlined herein and it may be displayed, e.g. on a touchscreen of external device 220. Hence, the data may be indicated to the patient. For example, the patient may be provided with an indication of the current pressure value, a (long-term) trend of pressure values, etc. Also, temperature and/or acceleration data based on the received temperature and acceleration values may optionally be displayed, possibly after adaptation for display by external device 220.

External device 220 may further be connected to the internet 240, e.g. wirelessly via Wi-Fi, 4G, 5G, etc. It may forward the pressure data (and optionally patient temperature and/or acceleration data) to an optional further device 260, e.g. a device (e.g. smartphone, tablet, etc.) of the physician supervising the patient, such that it may also be displayed 264, there. In particular, it may be displayed to the physician for analysis to find the right dose of medication and/or react to possible deviations of the pressure values from target values (e.g. during ongoing medication).

External device 220 may further forward pressure data (e.g. the arterial pressure values, possibly adapted based on barometric pressure values, or data based thereon) to remote system 250. Optionally, also the acceleration and/or temperature data may be forwarded to remote system 250 by external device 220. Remote system 250 may comprise one or more servers, databases, etc. In some examples, the further device 260 may retrieve the data from remote system 250 additionally or alternatively to receiving it from external device 220.

Remote system 250 may be configured with one or more algorithms to process the pressure data (and optionally the temperature and/or acceleration data). It may additionally be provided with further patient data, such as a hypertension diagnosis, a current medication plan (if any), etc. Remote system 250, based on the processing, may derive feedback 262, such as a recommended action (e.g. a suitable initial medication dose for mitigating the hypertension, a reminder to take a specific medication dose to mitigate the hypertension) or an alarm (e.g. in case of an unforeseen emergency situation such as in case of a rapid increase or decrease of pressure, for example; a suspected overdosing of the medication, etc.). It may also recommend actions based on an activity level of the patient (e.g. as determined based on the accelerometer data), e.g. recommend more sports activities in case of a low activity level, for example. For example, the one or more algorithms may be based on an artificial intelligence, that has been trained with historic patient data. Remote system 250 may provide feedback 262 to external device 220, for example, via the internet 240. The patient may then react accordingly, such that the patient is assisted with handling the hypertension. Also, feedback 262 may be provided to the further device 260, e.g. to also provide the alarm or recommended action to the physician. The corresponding alarm or recommended action may then also be displayed by the external device 220 (e.g. its touchscreen) and/or the further device 260. It may also be possible that other optical, acoustic and/or haptic elements of external device 220 or further device 260 are used to indicate the alarm or recommended action, such as a flashlight, an acoustic alarm or voice output, a vibration, etc. Apart from that, also text messages, phone calls, etc. may be used to indicate an alarm and/or recommended action.

For example, an alarm and/or recommended action (to take a prescribed medication, to change a prescribed medication, etc.) may be based on simple threshold value crossings. The thresholds may relate to pressure thresholds that may be different depending for example on an activity level (determined by external device 220 and/or remote system 250 based on the acceleration data, for example).

External patient device 220 and remote device 260 may be general purpose devices (e.g. smartphones, tablets, etc.) that are provided with the functionality as described herein by means of an application or program. It is understood that the relevant communication by patient device 220 and remote device 260 may be secure, e.g. encrypted.

In other examples, further device 260 may not require a dedicated application or program, but instead may access the display data 264 and the alarm and/or recommended action 262 via a web graphical user interface, accessible by an internet browser.

A system according to the present disclosure may be understood as comprising implantable pressure sensor 210 and external device 220 (and optionally relay device 230). Alternatively, it may comprise implantable pressure sensor 210 and further device 260 (configured as external device). Additionally, the systems may comprise remote system 250 and/or the respective additional further device 260 and/or external device 220.

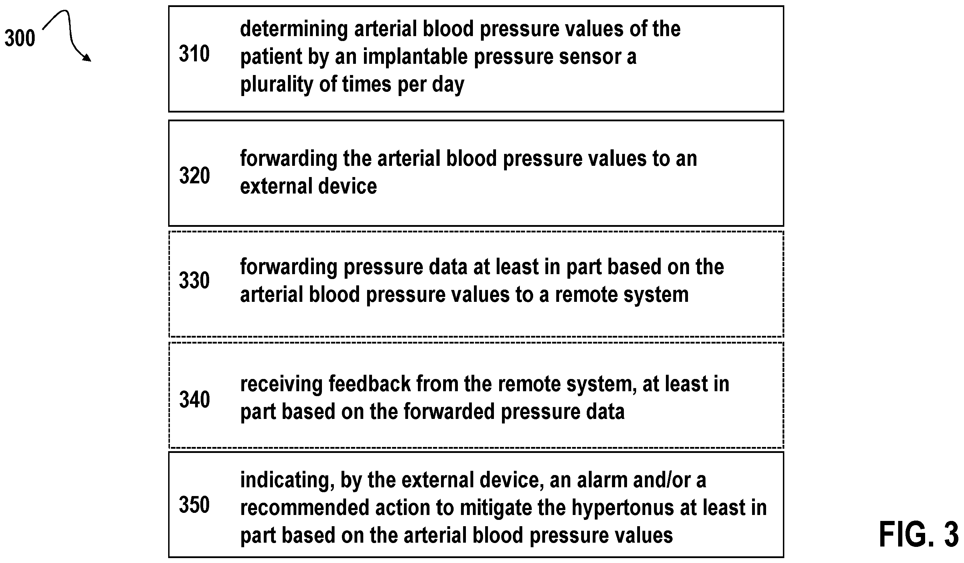

An exemplary method for continuously monitoring an arterial blood pressure of a patient is shown in Figure 3. The arterial pressure of the patient is determined by an implantable pressure sensor 210 a plurality of times per day 310. The determined data are forwarded 320 to an external device 220 and further forwarded 330 to a remote system 250. As described above the determined pressure data from the implantable device 210 may be corrected with the barometric pressure determined by the external device 220 to obtain the arterial pressure of the patient. Typically, the pressure data from the implantable device 220 corrected with the barometric pressure are forwarded to the remote system 250 in step 330. The data are analysed by the remote system 250 and a corresponding feedback is created 340. Depending on the feedback of step 340 an alarm and/or a recommended action to mitigate the hypertension.

It is noted that it may also be possible to combine the systems and methods described herein with one or more further implantable pressure sensors. For example, a further sensor may be implanted specifically to monitor aspects related to heart failure patients and/or patients having pulmonary hypertension, wherein the further sensor may be placed such as to acquire a pulmonary artery pressure, a pressure in a left atrium or a filling pressure towards the left ventricle, for example. Also, pressure data from further sensors may then be forwarded, processed for display, displayed, and/or analysed as described herein. Moreover, alarms and/or recommended actions may be derived also based on data from further sensors as described herein, specifically in order to mitigate heart failure and recommend adaptations to medications, for example.

## Claims

1. A method (100) for continuously monitoring an arterial blood pressure of a patient, comprising:
determining (120) arterial blood pressure values of the patient by an implantable pressure sensor (210) for a plurality of times per day;
forwarding (140) the arterial blood pressure values to an external device (220); and
indicating (160), by the external device, blood pressure values (264) at least in part based on the arterial blood pressure values.

2. The method of claim 1, further comprising adapting the arterial blood pressure values at least in part based on barometric pressure values (P).

3. The method of claim 2, wherein the barometric pressure values are determined by the external device.

4. The method of claim 3, further comprising the step of determining the proximity between the patient and the external device at the time of the determination of the barometric pressure value by the external device.

5. The method of claim 4, wherein step of determining the proximity between the patient and the external device at the time of the determination of the barometric pressure may comprise at least one of the following:
- comparing the motion profiles of the implant and the external device
- Signal exchange between implant and external device prior, during or after the determination of the barometric pressure.

6. The method of any of claims 1 to 5, further comprising:
determining patient temperature values and/or patient acceleration values by the implantable pressure sensor for a plurality of times per day;
forwarding the determined patient temperature values and/or patient acceleration values to the external device.

7. The method of any of claims 1 to 6, comprising determining arterial blood pressure values at different activity levels of the patient.

8. The method of any of claims 1 to 7, further comprising the step of forwarding, by the external device, pressure data based on the arterial blood pressure values to a remote system (250).

9. The method of claim 8, further comprising the step of receiving feedback (262) from the remote system based on the pressure data.

10. The method of any of claims 1 to 9, further comprising the step of indicating an alarm and/or a recommended action (262) by the external device at least in part based on the arterial pressure values.

11. The method of claim 10, wherein the alarm and/or recommended action is further based on at least one of: the patient temperature values and/or patient acceleration values of claim 4; the feedback of claim 7.

12. The method of any of claims 1 to 11, further comprising the step of comparing the determined arterial blood pressure values and/or barometric pressure values with a trend of previously determined arterial blood pressure values and/or barometric pressure values.

13. The method of any of claims 1 to 12, further comprising the step of filtering the determined arterial blood pressure values and/or barometric pressure values with a median filter, using previously and subsequently determined arterial blood pressure values and/or barometric pressure values.

14. A system (200) for continuously monitoring an arterial blood pressure of a patient, comprising:
an implantable pressure sensor (210) for determining arterial blood pressure values of the patient a plurality of times per day;
an external device (220) for receiving the arterial blood pressure values from the implantable pressure sensor; and
wherein the external device is configured to indicate blood pressure values (264) at least in part based on the arterial blood pressure values.

15. System (200) for assisting a patient having hypertension, comprising:
an implantable pressure sensor (210) for determining arterial blood pressure values of the patient a plurality of times per day;
an external device (220) for receiving the arterial blood pressure values from the implantable pressure sensor; and
wherein the external device is configured to indicate an alarm and/or a recommended action to mitigate the hypertension at least in part based on the arterial blood pressure values.

16. The system of claim 14 or 15, wherein the implantable pressure sensor (210) comprises an accelerometer (213) and/or a temperature sensor (214).

17. The system of any of claims 14 to 16, wherein the implantable pressure sensor is an active sensor.

18. Method (300) for assisting a patient having hypertension, comprising:
determining (310) arterial blood pressure values of the patient by an implantable pressure sensor a plurality of times per day;
forwarding (320) the arterial blood pressure values to an external device; and
indicating (350), by the external device, an alarm and/or a recommended action to mitigate the hypertension at least in part based on the arterial blood pressure values.

19. Method of claim 18, further comprising determining the arterial blood pressure values at different activity levels of the patient.
